Europäisches Patentamt

European Patent Office

Office européen des brevets

(11) Publication number: **0 187 858 B1**

# EUROPEAN PATENT SPECIFICATION

(45) Date of publication of patent specification: **08.07.92**

(51) Int. Cl.⁵: **C07K 7/02**, C07K 7/06, A61K 39/00, A61K 39/12, C12P 21/02, C12P 19/34

(21) Application number: **85903931.5**

(22) Date of filing: **23.07.85**

(86) International application number: **PCT/US85/01416**

(87) International publication number: **WO 86/00911 (13.02.86 86/04)**

(54) **PROTECTIVE PEPTIDE ANTIGEN CORRESPONDING TO PLASMODIUM FALCIPARUM CIRCUMSPOROZOITE PROTEIN.**

(30) Priority: **23.07.84 US 633147**

(43) Date of publication of application:
**23.07.86 Bulletin 86/30**

(45) Publication of the grant of the patent:
**08.07.92 Bulletin 92/28**

(84) Designated Contracting States:
**AT BE CH DE FR GB IT LI LU NL SE**

(56) References cited:
**EP-A- 0 166 410**
**EP-A- 0 175 261**
**EP-A- 0 192 626**
**WO-A-86/05790**

**SCIENCE, vol. 225, 10th August 1984, pages 628-629, Washington, US; V. ENEA et al.: "DNA cloning of Plasmodium falciparum circumsporozoite gene: amino acid sequence of repetitive epitope"**

(73) Proprietor: **NEW YORK UNIVERSITY**

70 Washington Square South
New York, NY 10012(US)

(72) Inventor: **ELLIS, Joan**
**415 East 52D Street**
**New York, NY 10022(US)**
Inventor: **ENEA, Vincenzo**
**500 East 63D Street**
**New York, NY 10021(US)**
Inventor: **NUSSENZWEIG, Ruth, S.**
**110 Bleeker Street**
**New York, NY 10012(US)**
Inventor: **NUSSENZWEIG, Victor, N.**
**110 Bleeker Street**
**New York, NY 10012(US)**

(74) Representative: **Patentanwälte Grünecker, Kinkeldey, Stockmair & Partner**
**Maximilianstrasse 58**
**W-8000 München 22(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

SCIENCE, vol. 225, 10th August 1984, pags 593-599, Washington, US; J.B. DAME et al.: "Structure of the gene encoding the immunodominant surface antigen on the sporozoite of the human malaria parasite Plasmodium falciparum"

SCIENCE, vol. 228, 24th May 1985, pages 958-962, Washington, US; J.F. YOUNG et al.: "Expression of Plasmodium falciparum circumsporozoite proteins in Escherichia coli for potential use in a human malaria vaccine"

SCIENCE, vol. 228, 24th May 1985, pages 996-999, Washington, US; W.R. BALLOU et al.: "Immunogenicity of synthetic peptides from circumsporozoite protein of Plasmodium falciparum"

SCIENCE, vol. 228, 21st June 1985, pages 1436-1440, Washington, US; F. ZAVALA et al.: "Rationale for development of a synthetic vaccine against Plasmodium falciparum malaria"

NATURE, vol. 305, 1st September 1983, pages 29-33, Macmillan Journals Ltd, London, GB; G.N. GODSON et al.: "Identification and chemical synthesis of a tandemly repeated immunogenic region of Plasmodium knowlesi circumsporozoite protein"

MOLECULAR AND BIOCHEMICAL PARASITOLOGY, vol. 5, no. 6, 1982, pages 391-400, Elsevier Biomedical Press, Amsterdam, NL; M. GOMAN et al.: "The establishment of genomic DNA libraries for the human malaria parasite Plasmodium falciparum and identification of individual clones by hybridisation"

## Description

The Government has rights in this invention based upon research support in the form of Grant No. 5RO1-AI-17429-03 from the Department of Health and Human Services and Grant No. AID-DPE-0453-C-00-2002-00 from the Department of State, Agency for International Development.

Background of the Invention

The present invention relates to an antigen suitable for providing protective immunity against malaria e.g. by incorporation into a vaccine. A formidable health problem in large areas of the world, malaria affects more than 150 million people in any given year. Of the four plasmodial species which cause malaria in humans, Plasmodium falciparum is responsible for most of the severe infections and the highest rate of mortality. Combating malarial infestations caused by P.falciparum has become more difficult due to the spread of drug-resistant organisms in many areas. The occurrence of severe epidemic outbreaks of this disease lends particular urgency to recent efforts to develop a malaria vaccine.

Under normal conditions, a malarial infection is initiated by the introduction of sporozoites into the bloodstream of the host through the bite of infected mosquitoes. Hence, inactivation of these sporozoites by the immune system of the host could completely block development of the infection. Several recent findings point to the feasibility of developing an antisporozoite vaccine. Sporozoites are highly immunogenic and are capable of eliciting a protective immune response in several host species, including man: see e.g. Cochrane, A.H. et al. Malaria, Vol. 3, J.D. Kreier, Ed. (Academic Press, New York 1980), pp. 163-202. The immunogenicity of sporozoites resides largely, if not exclusively, in a single antigen, the circumsporozoite (CS) protein (described in detail by F. Zavala, A.H. Cochrane, E.H. Nardin, R.S. Nussenzweig, V. Nussenzweig, J. Exp. Med. 157: 1947 (1983), which covers the entire parasite surface, as reported by M. Aikawa, N. Yoshida, R.S. Nussenzweig and V. Nussenzweig in Journal of Immunology, 126: 2494 (1981). Finally, the immunogenicity of the CS protein is restricted almost entirely to a single epitope which is identically or quasi-identically repeated several times in tandem: G.N. Godson, et al. Nature 305: 29 (1983); V. Enea et al., accepted for publication Proc. Nat'l. Acad. Sci. (1984).

Identification of the amino acid sequence of CS epitopes for all plasmodial species that infect humans is a prerequisite for the development of a human synthetic sporozoite vaccine.

Several monoclonal antibodies have been raised against the CS protein of Plasmodium falciparum sporozoites. These antibodies inactivate the parasites. Methods for obtaining such antibodies are well known in the art and have been disclosed by Nardin E. et al. in J. Exp. Med. 156: 20 (1982), and in U.S. Patent Application Serial No. 234,096 of Nussenzweig et al, filed February 12, 1981 the disclosure of which is incorporated herein by reference. (The disclosure of this application also incorporates by reference the entire disclosure of assignee's copending U.S. Patent Application Serial No. 574,553 filed January 27, 1984 of Nussenzweig, et al. entitled Protective Peptide Antigen).

These monoclonal antibodies against CS protein bind to a repeated epitope, which is common to different isolates of parasites obtained from different geographical areas. Such antibodies can be used to screen clones expressing peptides having or incorporating the amino acid sequence of the CS repetitive epitopes.

Antibodies against the sporozoite antigens have been shown to provide protective immmunity against the plasmodium species from which they were derived, in rodents, monkeys and in human volunteers. The sporozoite protective antigen is herein termed CS protein, or circumsporozoite protein, or sporozoite CS protein, these terms being deemed equivalent and used interchangeably. Assignee's copending U.S. Patent Application of Nussenzweig, Serial No. 234,096 filed February 12, 1981 discloses a vaccine based upon purified CS protein. Assignee's copending application Serial No. 574,553 discloses a peptide comprising an epitope of a sporozoite CS protein.

The results disclosed herein are based in part on techniques and concepts in the field of immunology. For convenience, certain terms commonly used in the art are defined herein. The term "immunochemical reaction" is used to denote the specific interaction which occurs between an antigen and its corresponding antibody, regardless of the method of measurement. Such a reaction is characterized by a non-covalent binding of one or more antibody molecules to one or more antigen molecules. The immunochemical reaction may be detected by a large variety of immunoassays known in the art. The terms "immunogenic" or "antigenic" are used here to describe the capacity of a given substance to stimulate the production of antibodies specifically immunoreactive to a substance when that substance is administered to a suitable test animal under conditions known to elicit antibody production. The term "protective antigen" refers to the ability of a given immunogen to confer resistance in a suitable host, against a given pathogen. The term "epitope", refers to a specific antibody binding site on an antigen. Macromolecular antigens such as proteins typically have several epitopes with distinctive antibody binding specificities. Different epitopes of the Same antigen are distinguishable with the aid of monoclonal antibodies which, due to their high degree of specificity, are directed against a single epitope. Two different monoclonal antibodies directed against different epitopes on the same antigen may bind the antigen without interfering with the other, unless the epitopes are so close together that the binding of one sterically inhibits the binding of the other. The term "immunodominant region" denotes an area of the antigen molecule which is mainly responsible for its antigenicity.

## Summary of the Invention

The present invention involves the discovery that the protective CS sporozoite antigens of P. falciparum possess an immunodominant region composed of four amino acids (proline-asparagine-alanine-asparagine) that are tandemly repeated at least 23 times. The repeat comprises 8 variants at the nucleotide level. Both asparagine codons, three of the four proline codons and two of the four alanine codons are employed. This repeated sequence has been shown to contain the epitope of the CS protein of Plasmodium falciparum. Analogs of the repeated peptide have been chemically synthesized and have been found to be immunochemically reactive with polyclonal antibody preparations against Plasmodium falciparum. In addition, monoclonal antibodies against CS proteins, which neutralize the infectivity of sporozoites in vitro, also react with the synthetic peptide. Vaccines made with three and six tandem repeats of the four amino acid sequence (12-MER and 24-MER peptides) confer immunity to P. falciparum sporozoites. Thus, these synthetic peptides exhibit the protective antigenic features of the P.falciparum CS protein.

## Detailed Description of the Invention

In the following description, the materials employed were commercially available, unless otherwise specified. Enzymes used in the cloning procedures were obtained from commercial sources. Restriction endonuclease reactions were carried out according to the manufacturer's instructions. Unless otherwise specified, the reaction conditions for other enzyme reactions were standard conditions used in the art, as described, for example, in Methods in Enzymology, (Vol. 68, R.Wu, Ed.) Academic Press, (1980). Unless otherwise specified, the abbreviations herein are standard abbreviations acceptable for publication in scientific journals normally used by those skilled in the art to publish their results, such as those cited herein.

Monoclonal antibodies to P.falciparum sporozoites were isolated from mouse ascites injected with hybridomas produced by fusing the spleen cells of P.falciparum sporozoite-hyperimmunized mice with NS1 myeloma cells as described in Nardin, E.H., et al J. Exp. Med. 156:20-30 (1982). The monoclonal antibody used to identify the clone expressing the protective peptide antigen of the present invention was prepared according to the procedures of Nardin, et al., supra, and designated "2A10."

In general outline, the experiments and conclusions following from the results thereof are set forth. The synthetic protein of the present invention was defined and initially secured by cloning a cDNA made from mRNA obtained from infected mosquitoes. A cDNA library was constructed from poly (A)$^+$ RNA derived from Plasmodium falciparum infected mosquitoes. Double-stranded cDNA was inserted at the PstI site of plasmid pBR322 using the dC -dG tailing method to generate recombinant plasmids that could express the inserts as a fusion protein with the beta-lactamase encoded by the vector. Bacterial host cells (LE 392 derived from E. coli K-12) were transformed and the resulting tetracycline resistant DNA molecules were screened for the expression of CS antigen using an in situ filter immunoassay.

Approximately 10,000 colonies were screened by an in situ radioimmunoassay with the monoclonal anti-sporozoite antibody (2A10) and a strongly positive clone, designated p277-19 was identified.

Extracts of the host bacterium LE392, harboring the plasmid p277-19 were then tested in a two-site immuno-radiometric assay by using monoclonal antibody 2A10 immobilized in plastic wells, and the same [$^{125}$I]-labelled antibody in the fluid phase: F. Zavala et al, Nature 229: 737 (1982).

The recombinant protein expressed by clone p277-19 is able to bind simultaneously both the immobilized and the radiolabelled antibody. This indicates that the recombinant protein, as the authentic CS protein, contains at least two epitopes which are recognized by the anti-CS monoclonal antibody 2A10.

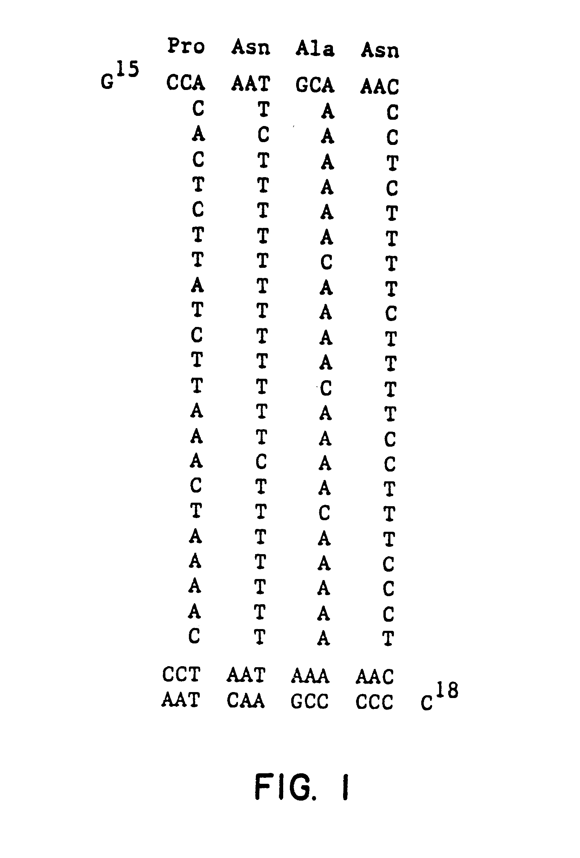

The nucleotide sequence of the p277-19 insert is illustrated in Fig. 1. In the protein encoded by this sequence, the amino acid sequence proline, asparagine, alanine and asparagine is repeated 23 times in tandem with no variations. This repetitive pattern of four amino acids is the shortest of the known CS protein repeats. The repeats of P. knowlesi and P. cynomolgi (Gombak strain), two simian malaria parasites, are twelve and eleven amino acids long, respectively, Godson, et al. Nature 305: 29 (1983); V. Enea et al. supra (1984).

Although neither the DNA nor the protein sequences of these repeated peptides are related to one another, certain similarities are apparent from an analysis of their amino acid composition. Thus, alanine and asparagine are present in the repeats of all known CS proteins; proline is present in P. knowlesi and P. falciparum; and glutamic acid and glycine are present in P. knowlesi and P. cynomolgi (gombak).

The present findings indicate that the immuno-dominant epitope of the CS protein of P. falciparum consists of a sequence of amino acids which does not appear to require further modification to be antigenic.

EXAMPLE I.

Preparation of Plasmodium falciparum RNA

RNA was prepared from the thoraces of Anopheles balabacensis mosquitoes infected with Plasmodium falciparum of the Thai K-I stain. The collected thoracic tissue (from 1837 mosquitoes) was homogenized in 10 volumes of 4 M guanidine isothiocyanate (pH 5.0) and 0.1M 2-mercaptoethanol (Liu et al, Proc. Nat'l Acad. Sci. (USA) 76:4503 1979; Ellis et al, Nature 302:536 (1983). The homogenate was centrifuged at 9,000 rpm for three minutes in a Sorval (RC2-6) centrifuge. The supernatant was then layered over 0.2 volumes of 5.7M cesium chloride and 0.1 EDTA (pH 6.5) and centrifuged in an SW-41 rotor at 28K for 16 to 20 hours at 20°C. The RNA pellet was resuspended in 7.5M guanidine hydrochloride in 25 mM sodium citrate (pH 7.0) with 5 mM beta-mercaptoethanol. The RNA was precipitated by adding one fortieth volume, I M acetic acid and one half volume of 95% ethanol at -20°C for two to three hours (Chirgwin, et al. Biochem. 18:5294 1979). This was followed by a second precipitation in 0.3M sodium acetate (pH5) and 2.5 volumes of 95% ethanol, overnight at -20°C. Following centrifugation the RNA pellet was resuspended in water and stored at -70°C.

EXAMPLE II.

Purification of the Poly(A)[+] RNA

Poly (A)[+] RNA was prepared according to the method of Aviv and Leder Proc. Nat'l Acad. Sci. (USA) 69:1408 (1972). The RNA was heated at 68°C for 10 minutes, then chilled on ice for 5 minutes. After warming the RNA sample to room temperature, binding buffer was added to a final concentation of 0.5M sodium chloride, 0.01M Tris-HCl (pH 7.4) and 0.01M EDTA (pH 7.0). The RNA was cycled 3-5 times through an oligo(dT) cellulose (Collaborative Research, Inc., Waltham, Mass.) column with a bed volume of 0.2 - 0.4 ml. The poly(A[+]) RNA was eluted from the column with sterile water at room temperature. The RNA was recovered by precipitation with ethanol, and stored in water at -70°C.

Prior to the cDNA synthesis, 1.5 micrograms of the poly(A)[+] RNA was mixed with 75 nanograms of rabbit globin mRNA (Bethesda Research Laboratories, (BRL), Bethesda, Md.) extracted first with phenol and chloroform (1:1 v/v) and then with chloroform. The RNA was precipitated in 0.3 M sodium acetate and 2 and $^1$/2 volumes of 95% ethanol. The pellet was resuspended in six microliters of water and then stored at -70°C.

EXAMPLE III.

Construction of the cDNA Library from Poly (A)[+] RNA

The first and second strands of the cDNA were synthesized by a modification of the procedure of Okayama and Berg, Molecular and Cellular Biology 2:161 (1982). Approximately 1.5 micrograms of P. falciparum poly(A)[+] RNA mixed with 75 nanograms of rabbit globin mRNA (Bethesda Research Laboratories), were incubated in a 30 microliter reaction volume containing 50 mm Tris-HCl (pH 8.3), 50 mm KCl, 8 mm MgCl$_2$, 2 mm dithiothreitol, 30 micrograms/ml oligo-dT$_{(12-18)}$ cellulose (Collaborative Research), 100 micrograms/ml Actinomycin-D (Sigma Chemical Co., St. Louis, Mo.), 100 micrograms/ml BSA (bovine serum albumin), 0.25 mM dATP, 0.5 mM dCTP, 0.5 mM dGTP, 0.5 mM dTTP, 50 x $10^{-6}$ Ci alpha-[$^{32}$P] dATP (specific activity 3,000 curies per millimole) and 120 units of reverse transcriptase (BRL) at 42°C for 2 hours.

The reaction was stopped by extraction with phenol and chloroform (1:1 v/v), then with an equal volume of chloroform and precipitated two times with 2 M ammonium acetate and ethanol. The pellet was washed with 80% ethanol, dried by dessication under vacuum, and resuspended in 46.9 microliters of water.

The second strand was synthesized in a 65 microliter reaction volume containing 20 mM of Tris-HCl (pH 7.4), 4 mM of magnesium chloride, 10 mM $(NH_4)_2SO_4$, 0.1 mM of KCl, 50 micrograms/ml BSA, 0.3 mM nicotinamide adenine dinucleotide (NAD) oxidized (Sigma), 0.1 mM each of the deoxynucleotide triphosphates (dATP, dCTP, dTTP, dGTP[1/]), one unit DNA Polymerase I (Boerhinger Mannheim Biochemical, Indianapolis, Indiana), 1.5 units RNAase H (BRL), 1 unit E.Coli ligase (P.L. Biochemical). The reactants were first incubated at 15°C for one hour, then at room temperature for one hour. Again, the reaction was stopped by extraction, first with phenol/ chloroform (1:1) and then with an equal volume of chloroform. The mixture was precipitated once with 2M ammonium acetate and ethanol and the pellet washed with 80% ethanol, dried and resuspended in 4.5 microliters of water.

The second strand synthesis reaction was completed with $T_4$ DNA polymerase (BRL). The double stranded cDNA was incubated in 50 mM Tris-HCl (pH 8.0), 6 mM magnesium chloride, 25 mM KCl, 0.1 mM each of dATP, dCTP, dGTP and dTTP and 3.5 units of $T_4$ DNA Polymerase at 37°C for 30 minutes. The reaction was stopped by the addition of 25 mM EDTA. The reaction mixture was extracted with phenol and chloroform (1:1 v/v), and then with an equal volume of chloroform, followed by three washes with ether. The double-stranded cDNA was then precipitated with 0.5 M NaCl and 10% PEG (polyethylene glycol, average molecular weight 8,000) at 4°C overnight. The double-stranded cDNA was tailed with deoxycytidine residues according to Roychoudhury, et al. Nucl. Acids Res., 3, 101 (1976); Land et al. Nucl. Acids Res. 9:2251 (1981). Double-stranded cDNA (30 to 60 nanograms) were incubated in a 25 microliter reaction volume with 0.1 M potassium cacodylate (pH 7.0), 0.5 mM dCTP, 0.1 mM DTT and 2 mM $CoCl_2$ at 37°C for five minutes. Ten units of terminal deoxynucleotidyl transferase (Enzo Biochemical, Inc.) were added and the mixture was incubated at room temperature for 1 minute. The reaction was stopped by the addition of EDTA to 10 mM. Two micrograms of yeast tRNA were added and the mixture was extracted twice with phenol/chloroform (1:1) and once with chloroform, and then precipitated with 2M ammonium acetate and ethanol.

The deoxy(C)-tailed double-stranded cDNA was resuspended in 60 microliters annealing buffer (10 mM Tris-HCl, pH 7.4, 100 mM NaCl, and 1 mM EDTA). The concentration was estimated to be 0.6 to 3 micrograms/microliter. The tailed cDNA was annealed (using the method of Land, et al. supra 1981) to PstI-cut and deoxy(G)-tailed pBR 322 (New England Nuclear) at varying ratios to determine the optimal ratio of insert to vector.

All of the pilot annealings were performed at a concentration of 250 nanograms pBR322/ml in a 200 microliter reaction volume by mixing 50 ng of pBR322 with 20, 8.0, 4.2 and 3.3 microliters of the tailed double-stranded cDNA. The 20 and 4.2 microliter pilot reactions yielded the maximum number of colonies and therefore were scaled up to make larger preparations for transformation.

## EXAMPLE IV

### Transformation of Host Cells

E. coli LE 392 cells were used as the bacterial host (P. Leder, et al. Science 196:175 (1977)). This is a variant of the E. coli K-12 strain. However, transformation may also be carried out in other host cells such as, DH1 available from the E. Coli Genetic Stock Center, Yale Univ. (CGSC No. 6040).

The host cells were transformed by the recombinant plasmids using a modification of the procedure of Hanahan et al. J. Mol. Biol., 166: 557-580 (1983). 2.5 nanograms of the hybrid plasmid were added to 210 microliters of competent LE 392 cells. The mixture was incubated on ice for 30 minutes, heat shocked at 42°C for 90 seconds and placed on ice for 1 to 2 minutes. 800 microliters of SOC (2% Bactotryptone (Difco Detroit, Mich.), 0.5 % yeast extract, 10 mM NaCl, 2.5 mM KCl, 10 mM $MgCl_2$, 10 mM $MgSO_4$, 20 mM glucose) were added and the reaction was incubated at 37°C shaking at 225 rpm for one hour. The cells were centrifuged at 2,000 rpm for 10 minutes and resuspended in 0.4 milliliters SOB without magnesium (2% Bactotryptone, 0.5% yeast extract, 10 mM NaCl, 2.5 mM KCl) and spread on two Hanahan plates (1% Bactotryptone, 0.95% yeast extract, 10 mM NaCl, 1.5% Bacto agar) with 12.5 micrograms/ml of tetracycline (Sigma). The transformation efficiency was approximately $10^5$ transformants per microgram of annealed DNA.

## EXAMPLE V

1/ This and all dNTP's were from P.L. Biochem., Milwaukee, Wisc.

Screening for the cDNA Library

The cDNA library was screened by a modification of the in situ radioimmunoassay as described by Helfman, et al. Proc. Nat'l Acad. Sci., (U.S.A.) 80: 31-35 (1983), as described by Enea et al, supra.

The bacteria were transferred onto 82 mM nitrocellulose filters (Millipore HATF Millipore, Bedford, Mass.). Replica filters were made and regrown on the tetracycline plates described above, at 37°C.

The bacterial colonies were lysed by placing the open petri dishes over 1 ml of chloroform for 15 minutes. The filters were then placed in individual petri dishes or pooled in trays containing 50 mm Tris-HCl (pH 7.5), 150 mM NaCl, 2 mM magnesium chloride, 0.1 mM PMSF (phenylmethylsulfonylfluoride, BRL) 3% BSA, 40 micrograms/ml lysosome, 1 microgram/ml, DNAase I and gently agitated at room temperature for 1 to 2 hours. The filters were rinsed in 50 mM Tris-HCl (ph 7.5), 150 mM NaCl for 1 to 2 hours and then incubated for 15 to 30 minutes in 50 mM Tris-HCl(pH 7.5), 150 mM NaCl, and 3% BSA. The filters were then incubated in a 50 ml volume with 50 X 10$^6$ cpm [$^{125}$I)-labelled monoclonal antibody 2A10 in 150 mM NaCl, 3% BSA with gentle rocking at room temperature overnight. The filters were washed extensively with 150 mM NaCl, 0.1% NP40 (Sigma), and 50 mM Tris-HCl (pH 7.5) air dried and mounted for autoradiography.

After screening approximately 10,000 colonies, one was found to react with the monoclonal antibody 2A10.

The clone was purified by streaking on LB plates (10% Bactotryptone, 50% yeast extract, 170 mM NaCl, 1.5% Bacto agar) containing 12.5 micrograms/ml of tetracycline.

A single colony was picked and tested by both the in situ radioimmunoassay procedure described above and the two-site radioimmunoassay (Ellis et al. Nature, Vol. 302: 536-538) (1983). In this procedure antibody 2A10 was adsorbed to the wells of a microtiter plate. Crude lysates of the bacterial clones to be tested were added to the wells and incubated for sufficient time to allow the immunoreactive protein present in the lysate to bind to the adsorbed monoclonal antibody. The wells were then washed to remove any contaminating proteins and radiolabelled monoclonal antibody 2A10 was added. The labelled antibody attached to the antigenic protein that is already bound to the surface of the microtiter well by the first monoclonal antibody. Extracts of LE 392 harboring the plasmid scored positive in this assay.

EXAMPLE VI

Nucleotide Sequencing of Clone p277-19

Plasmid DNA was prepared from LE392 (p277-19) using a modification of the method of Birnbaum et al. Nucleic Acid Research 7: 1515 -1523 (1979). Briefly, bacterial cells were grown in LB medium (containing 10g Bactotryptone, 5g Bacto yeast extract, 10g NaCl [adjusted to pH 7.5 with NaOH] per liter) either to saturation or to an optical density of OD-600 nm of approximately 0.4 in which case chloramphenicol was added to 0.17 mg/ml. The cultures were incubated by centrifugation and resuspended in approximately 20 volumes of 50mM glucose. 25mM Tris-HCl (pH 8), 10mM EDTA and 2 volumes of 0.2N NaOH and 1% SDS were added. After incubating the suspension on ice for 10 minutes, 1.5 volume of 5M potassium acetate (pH 4.8) was added. Following a 10 minute incubation on ice, the sample was centrifuged at 8,000 rpm for 60 minutes in a fixed angle Sorval rotor and the supernatant was collected and combined with 0.6 volumes of isopropanol. The precipitate was then resuspended in 10 mM Tris, 10 mM EDTA (pH 8.0) treated with RNase A (BRL; 20 micrograms/ml) and RNase T1 (BRL; 1 unit/ml) at 37°C for 45 minutes.

Carbowax 8,000 (Dow Chemical Co., Midland, Mich.) and NaCl were added to 10% w/v and 0.4M respectively and the sample was incubated at 4°C overnight. The preparation was then centrifuged at 8,000 rpm for 10 minutes and the pellet resuspended in 10 mM Tris (pH 8), and 1 mM EDTA, extracted with phenol/chloroform (1:1 v/v) and precipitated with ethanol.

Physical mapping of p277-19 with restriction enzymes MspI, HinfI, ScaI, BglI, PstI, AluI and RsaI (from BRL and New England Biolabs) revealed that the plasmid had suffered a deletion from approximately nucleotide 3350 to nucleotide 3608 on the standard pBR322 map (Sutcliffe, J.G., Cold Spring Harbor Sympos. Quant. Biol., 43:77-90 (1979)). As a result of this deletion, the PstI site 3' to the insert was missing and the HinfI site at nucleotide 3362 was very close to the 3' end of the insert. The physical sequence map of the vector 5' to the insert was unaltered. These findings influenced the selection of the technique for sequencing the insert as described below.

Six micrograms of the plasmid DNA were digested for 2 hours at 37°C with 24 units of MspI (New England Biolabs, Beverly, Mass.) in a 35 microliter reaction volume containing 10 mM Tris-HCl (pH 7.5), 10 mM magnesium chloride and 1 mM DTT. The digested plasmid DNA was fractionated on a 1.2% low melting agarose (International Biotechnologies, Inc., New Haven, Conn.) gel. The largest fragment, approximately 700 base pairs in length, which was determined to contain DNA insert (via the physical mapping described above), gel by melting the agarose slice at 70°C, followed by three sequential phenol extractions, one chloroform extraction and 2 cycles of precipitation in ethanol containing 2M ammonium acetate. The DNA was resuspended in 10 microliters of water and stored at -20°C.

Approximately 1 microgram of the gel purified p277-19 DNA was digested in a 10 microliter reaction volume with six units of Hinfl (BRL) at 37°C for one hour in Hin buffer (10 mM Tris-HCl, pH 7.5, 10 mM MgCl$_2$, 50 mM NaCl) and 1 mM DTT (dithiothreitol). The reaction was stopped by heating at 65°C for 10 minutes.

The Hinfl-digested DNA was then end-labelled in a 20 microliter reaction volume by adding each of dGTP, dTTP, dCTP to 50 mM and 30 x10$^{-6}$ Ci alpha-[$^{32}$P]-dATP (3,000 Ci/millimole) in Hin buffer (described above) with one mM DTT and two units of the Klenow fragment of E. coli DNA Polymerase I (Boerhinger-Mannheim) at room temperature for 15 minutes. Two microliters of 0.5 mM dATP were added and the incubation continued at room temperature for 10 minutes. The reaction was stopped by heating at 65°C for 10 minutes.

The end-labelled DNA fragments were fractionated in a 2% low melting temperature agarose mini gel. The fragments were electroeluted from the gel by cutting out a small well in front of the leading edge of the two DNA bands, filling the wells with approximately 35 microliters running buffer(0.04M Tris-acetate, 0.002 M EDTA) and continuing electrophoresis with four 45-60 second pulses (60 volts). The buffer in the wells was collected and the wells were refilled between each pulse of current.

Six micrograms of salmon sperm DNA (Sigma) were added to the DNA fragments and the mixture was extracted once with phenol and chloroform (1:1 v/v) and once with chloroform, followed by precipitation with 2M ammonium acetate and ethanol.

The precipitated DNA was resuspended in 53 microliters of water and sequenced according to the method of Maxam and Gilbert, Methods in Enzymology, Vol. 65, 499-560 (1980). The details of this method are set forth in Table I, which is based on a table of Maniatis, et al., "Recombinant DNA: A Cloning Manual" Cold Spring Harbor (1980).

The p277-19 DNA fragment encoded a peptide which contained a series of tandem amino acid repeats. The repetitive unit of the peptide was four amino acids in length and consisted of proline, asparagine, alanine and asparagine repeated 23 times in tandem. The nucleotide sequence of the DNA fragment is illustrated in Fig. 1. The sequence is aligned as a matrix with the reading frame in register with that of the beta-lactamase. The sequence was derived according to the method of Maxam and Gilbert supra using the Hpa II site 5' to the PstI insert in pBR322 and a Hinfl site 3' to the insert as labelling sites. Due to the 300 base pair deletion in the pBR322 on the 3' side of the insert, the Hinf I site has been brought to within 10 base pairs of the 3' end of the d(C)tailed cDNA insert.

TABLE I.  SUMMARY OF BASE-SPECIFIC REACTIONS FOR SEQUENCING END-LABELLED DNA

| | G | G & A | T & C | C | A C |
|---|---|---|---|---|---|
| | | | | | 1 mg of salmon sperm DNA in each |
| Mix | 200 μl DMS buffer<br>10 μl [$^{32}$P]DNA | 10 μl H$_2$O<br>10 μl [$^{32}$P]DNA | 10 μl H$_2$O<br>10 μl [$^{32}$P]DNA | 15 μl 5 M NaCl<br>10 μl[$^{32}$P]DNA | 100 μl { 2 N NaOH<br>1 mM EDTA<br>5 μl[$^{32}$P]DNA |
| Chill to | 0°C | 0°C | 0°C | 0°C | Heat to 90°C 3-4 min. |
| Add | 1 μl DMS | 25 μl formic Acid | 30 μl HZ | 40 μl HZ | 150 μl 1 N acetic acid<br>5 μl tRNA (1mg/ml)<br>750 μl 95% ethanol |
| Incubate | 20°C, 2-3 min. | 20°C, 5 min. | 20°C, 8 min. | 20°C, 12 min. | |
| Add | 50 μl DMS stop<br>750 μl ethanol | 200 μl HZ stop<br>750 μl ethanol | 200 μl HZ stop<br>750 μl ethanol | 200 μl HZ stop<br>750 μl ethanol | |
| Store | -70°C, 10-15 min. | -70°C, 10-15 min. | -70°C, 10-15 min. | -70°C, 10-15 min. | -70°C, 10-15 min. |
| Centrifuge | 10 min. | 10 min. | 10 min. | 10 min. | 10 min. |
| To pellet add | 250 μl 0.3 M NaAc<br>750 μl ethanol | 250 μl 0.3 M NaAc<br>750 μl ethanol | 250 μl 0.3 M NaAc<br>750 μl ethanol | 250 μl 0.3 M NaAc<br>750 μl ethanol | 250 μl 0.3 M NaAc<br>750 μl ethanol |
| Store | -70°C, 10-15 min. | -70°C, 10-15 min. | -70°C, 10-15 min. | -70°C, 10-15 min. | -70°C, 10-15 min. |

EP 0 187 858 B1

## TABLE I. CONTINUED

| | G | G & A | T & C | C | A C |
|---|---|---|---|---|---|
| Centrifuge | 10 min. | 10 min. | 10 min. | 10 min. | 10 min. |
| Rinse pellet with 70% ethanol | 70% ethanol | 70% ethanol | 70% ethanol | 70% ethanol | 70% ethanol |
| Vacuum dry | | | | | |
| To pelled add | 100 µl 1.0 M piperidine | 100 µl 1.0 M piperidine | 100 µl 1.0 M piperidine | 100 µl 1.0 M piperidine | 100 µl 1.0 M piperidine |
| Heat to | 90°C, 30 min. | 90°C, 30 min. | 90°C, 30 min. | 90°C, 30 min. | 90°C, 30 min. |
| Lyophilize | | | | | |
| Add | 20 µl $H_2O$ | 20 µl $H_2O$ | 20 µl $H_2O$ | 20 µl $H_2O$ | 20 µl $H_2O$ |
| Lyophilize | | | | | |
| Add | 10 µl $H_2O$ | 10 µl $H_2O$ | 10 µl $H_2O$ | 10 µl $H_2O$ | 10 µl $H_2O$ |
| Lyophilize | | | | | |
| Add | 10 µl loading buffer | 10 µl loading buffer | 10 µl loading buffer | 10 µl loading buffer | 10 µl loading buffer |
| Vortex | | | | | |
| Heat to | | | | | |
| Chill in Ice | 90°C, 1 min | 90°C, 1 min | 90°C, 1 min | 90°C, 1 min | 90°C, 1 min |
| Load onto Gel | | | | | |

Reactions should be carried out in siliconized Eppendorf tubes.

## EXAMPLE VII

Presence of Repetitive Epitopes in the Immunodominant Region of CS Proteins of P. falciparum.

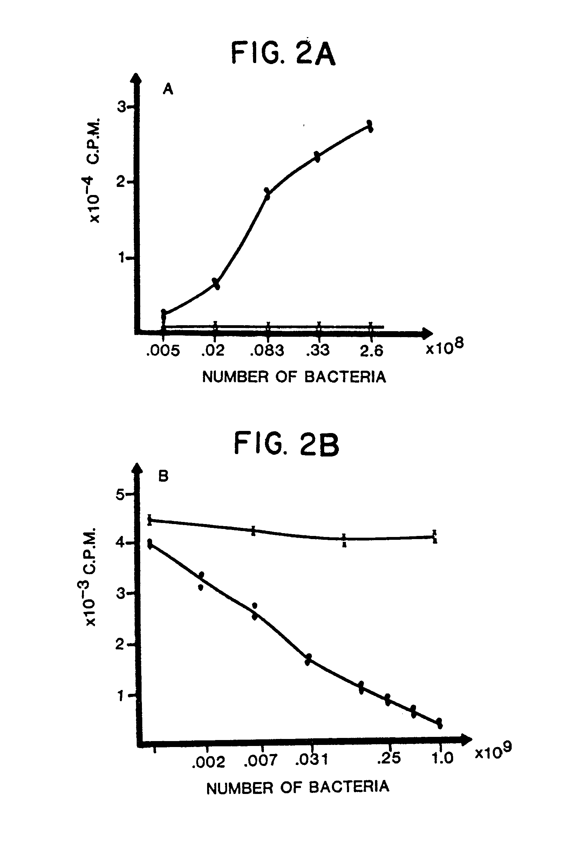

The presence of repetitive epitopes in P. falciparum CS proteins was confirmed by performing a two-site immunoradiometric assay with a single monoclonal antibody. This is illustrated in Figure 2A and 2B.

In this assay wells of flexible microtiter plates (Dynatech Inc.) were coated with 20 micrograms/ml anti-Plasmodium falciparum monoclonal antibody (2A10). After repeated washes with phosphate buffered saline

10

EP 0 187 858 B1

containing 1% bovine serum albumin, the wells were incubated with two fold serial dilutions of lysates of E. coli LE 392, containing plasmid p277-19 or E. coli LE 392 containing the pBR322 vector. Following a two hour incubation at room temperature, the wells were washed and 30 microliters of [$^{125}$I]-labeled monoclonal antibody 2A10 (1 X 10$^5$ cpm; specific activity 2 x 10$^7$ cpm/microgram) were added. After an incubation for one hour at room temperature, the wells were washed with PBS-Tween 20-BSA, dried and counted in a gamma counter. Lysates of E. coli LE 392 containing plasmid p277-19 were also tested using monoclonal antibody 2A10 coated plates and an unrelated [$^{125}$I]-labeled monoclonal antibody (X-X). As illustrated in Fig. 2A, the recombinant protein expressed by clone p277-19 simultaneously binds both the immobilized and the radiolabeled antibody. This indicates that the recombinant protein, like the authentic CS protein, contains at least two epitopes which are recognized by the anti-CS monoclonal antibody 2A10.

EXAMPLE VIII

Inhibitory Effect of Bacterial Extracts Made from LE 392 (p277-19) of the Binding of Labeled Monoclonal Antibody to the Epitopes of Authentic P. falciparum CS Proteins

The following inhibition assay was performed. 10 microliters (5 X 10$^4$ cpm) of [$^{125}$I]-labeled monoclonal antibody 2A10 were incubated with 30 microliters of two fold serial dilutions of lysates of E. coli LE 392 containing plasmid p277-19 (O-O) or E. coli LE 392 containing the pBR322 vector (X-X). Following a thirty-minute incubation at room temperature, 30 microliters of these mixtures were transfered into microtiter plates previously coated with an extract of P. falciparum sporozoites (Zavala et al., J. Exp. Med. 157:1947 (1983)). After a one hour incubation period the wells were washed, dried and counted in a gamma counter. The results of this inhibitory assay are illustrated in Fig 2B. The results show that bacterial extracts made from LE 392 containing the plasmid p277-19 inhibit the binding of labeled monoclonal antibody to the epitopes of native P. falciparum CS proteins. The specificity of this reaction was confirmed by a further experiment in which it was shown that cell extracts of p277-19 did not inhibit the binding of an anti-Plasmodium berghei monoclonal antibody to the corresponding CS protein. These data show that the recombinant protein encoded by p277-19 exhibits the antigenic feature of the P. falciparum CS protein.

EXAMPLE IX

Amino Acid Sequence of the 4-Amino Acid Repeat

The nucleotide sequence of the p277 19 insert was derived according to the method of Maxam and Gilbert, Proc. Nat'l. Acad.(USA) 74:560 (1977) using the Hpa II site 5' to the PstI site insert in pBR322 and a Hinfl site 3' to the insert as labelling sites. The nucleotide sequence of the p277-19 insert is illustrated in Fig. 1. The method followed is described in detail in Table I.

The deduced amino acid sequence of the four amino acid repeat set forth below is based upon translation of the nucleotide sequence in the correct reading frame: Pro-Asn-Ala-Asn. (All sequences are expressed from the end nearest the NH$_2$ terminus on the left to the end nearest the -COOH terminus on the right.)

The four amino acid sequence is repeated twenty-three times in tandem. However, at the nucleotide level, the repeats in p277-19 consist of eight variants. Both of the asparagine codons, three of the four proline codons, and two of the four alanine codons are used (Fig. 1). This repetitive pattern of four amino acids is shorter than any of the three known CS protein repeats. The repeats of P. knowlesi and P. cynomolgi (Gombak strain), two simian malaria parasites, are twelve and eleven amino acids long, respectively (Godson, et al. Nature 305:29 (1983); V. Enea et al. PNAS submitted (1984).

Although neither the DNA nor the protein sequences of these three sets of repeats exhibit extensive homology, they have similarities in their amino acid composition. Alanine and asparagine are present in the repeats of all three CS proteins; proline is present in P. knowlesi and P. falciparum; and glutamic acid and glycine are present in P. knowlesi and P. cynomolgi (Gombak).

The CS protein of P. falciparum appears to be encoded by a single copy gene based on the results of genomic DNA mapping experiments. In outline, the genomic clone was mapped as follows:

P. falciparum DNA obtained from blot stages was digested with restriction enzymes (including EcorI, BamhI, HindIII, BgIII, SalI, XhoI) fractionated on agarose gel, transferred to a nitrocellulose filter, hybridized with [$^{32}$P]-labeled p277-19 and autoradiographed. This procedure permits the determination of the sizes of the P. falciparum DNA (generated by all the above restriction fragments] that bears homology to the radioactive probe. Specifically, the SalI digest generated a fragment of approximately 7,000 nucleotides that hybridized with the probe. Since a fragment of this size was significantly smaller than the bulk of the fragments generated by Sall, a size-fractionation of Sall-digested DNA was undertaken to obtain the 7,000 nucleotide fragment generated by Sall which was expected to constitute a significant enrichment for the CS-gene.

Sall-digested DNA was fractionated on a sucrose gradient (10-40% w/v in 1M NaCl, 2mM Tris-HCl (pH 8) and 5mM EDTA; SW-41) at 38,000 rpm at 20°C for 16.5 hours. The fractions were collected and aliquots were hybridized to [$^{32}$P]-labelled p277-19.

The fraction that contained the CS sequence was ligated to Sall-digested EMBL4-DNA. (EMBL4 is a derivative of phage lambda; other Sall-digested phage lambda DNA vectors could have been employed, such as Charon 28 obtainable from BRL.)

The ligate was packaged in vitro (packaging extracts and protocols are commercially available from BRL and other sources) and plated on LE 392. The resulting plaques were screened with [$^{32}$P]-labelled p277-19. Two independent positive plaques were thus identified.

Characterization of the isolates is conducted by well-known techniques and includes physical mapping of the phages, subcloning of specific DNA fragments into plasmid vectors, determination of the DNA sequence of these fragments and, if necessary, mapping experiments with the messenger RNA of the P. falciparum CS protein. Using this procedure, the gene coding for the entire CS-protein of P. falciparum is isolated and sequenced.

EXAMPLE X

Synthesis of Peptides Having the Repeating Amino Acid Sequence

To confirm that the preceding amino acid sequence contains the immunoreactive site, a corresponding synthetic peptide has been synthesized using solid phase resin synthesis (Marglin, H. and Merrifield, R. B., Ann. Rev. Bio. Chem. 39:841-866 (1970). The general steps of the peptide synthesis techniques used herein are well known. The synthesis was carried out using a benzhydrylamine (BHA) resin on an automated synthesizer controlled by a computer using a program based on that of Merrifield, R. B., Fed. Proc. 21:412 (1962); J. Chem. Soc. 85:2149, (1963). The four amino acid repeat was assembled on the benzhydrylamine resin. The tandem repeat was assembled by the sequential addition of protected amino acids in the same order as the four amino acid repeat, using the method described above. Amino acid composition and sequence analysis performed by automated Edman degradation confirm that the peptide had been correctly synthesized. A 12-MER peptide was thus synthesized which consisted of three sequential repeats of the minimum repeating unit (Pro-Asn-Ala-Asn).

To confirm that the correct epitope has been obtained, rabbits are immunized with a peptide consisting of three and six tandem repeats of the four amino acids coupled to a carrier (bovine gamma globulin in complete Freund's adjuvant). Four weeks after the injection, the rabbits are bled and their serum assayed for the presence of antibodies against the tandemly repeated peptides and against extracts of P. falciparum sporozoites. The results show that the animals produce high titers (greater than 1:1000) of antibodies to the native CS protein present in the parasite extracts.

EXAMPLE XI

Inhibition of the Binding of Monoclonal Antibody
To Authentic P. falciparum Antigen by the
Synthetic Peptide

The antigenicity of a synthetic 12-amino acid peptide consisting of a 3X tandem repeat of the minimum repeating unit (Pro-Asn-Ala-Asn) of the P. falciparum CS protein was confirmed by a direct radioimmunoassay, as follows:

P. falciparum sporozoite extract was used to coat the bottom of microtiter well plates (as previously described). Unbound native antigen was removed by washing and the wells were filled with serial dilutions of PBS-BSA containing serial dilutions of the synthetic 12-amino acid peptide having the sequence (Pro-Asn-Ala-Asn-Pro-Asn-Ala-Asn-Pro-Asn-Ala-Asn.) Control wells were filled with serial dilutions of PBS-BSA containing the synthetic 12-amino acid peptide representing the epitope of P. knowlesi, i.e. (Gln-Ala-Gln-

EP 0 187 858 B1

Gly-Asp-Gly-Ala-Asn-Ala-Gly-Gln-Pro). Saturation amounts of [$^{125}$I]-labelled monoclonal antibody 2A10 were then added to the wells (8 x 10$^4$ cpm) and allowed to bind. After removal of the supernatant residual radioactivity was measured with a gamma counter. The results are shown in Table II.

TABLE II

| Well No. | 1 | 2 | 3 | 4 | 5 | 6 |
|---|---|---|---|---|---|---|
| (1) P. falciparum 12-peptide (micrograms/ml) | 500 | 50 | 5 | 0.5 | 0.05 | 0.005 |
| (2) Residual Radio-activity of (1) (cpm) | 194 | 297 | 1590 | 4990 | 6092 | 6271 |
| (3) Non-Specific Antigen (P. knowlesi 12-peptide) (micrograms/ml) | 500 | 50 | 5 | 0.5 | 0.05 | 0.005 |
| (4) Residual Radio-activity of (3) (cpm) | 5179 | 5838 | 6170 | 6409 | 6174 | 6181 |

Control on wells coated with BSA alone without sporozoite extract showed a residual radioactivity of 27-58 cpm.

The above results show that the monoclonal antibody recognizes and quantitatively binds to the synthetic 12-amino acid peptide.

EXAMPLE XII

Recognition of the Synthetic Peptide By Monoclonal Antibodies to P. falciparum CS-Protein

Another immunoradiometric assay was used to show that the synthetic 12-amino acid peptide is recognized by several antibodies to P. falciparum CS protein. The antibodies used are designated 2A10, 1E9, 3D6, and 2C11.

A synthetic 12-MER peptide (three repeats of the pro-asn-ala-asn peptide) (20 micrograms/ml) was bound to the bottom of microtiter wells as previously described. The wells were saturated with BSA.

Serial dilutions of each type of unlabelled monoclonal antibody preparation (10 micrograms/ml) in serial dilution were introduced into separate 12-MER coated wells, and sufficient time was allowed for the antibody to bind to the coat.

Finally, after washing the wells, saturation amounts of affinity-purified, radiolabeled goat antimouse IgG were also introduced into the wells and allowed to bind to the monoclonal antibodies bound to the peptide coat. The wells were then washed and residual radioactivity was measured in a gamma counter. The results are summarized in Table III below.

Unlabelled monoclonal antibodies to P. knowlesi, BSA coated wells (in the absence of anti-P. falciparum monoclonal antibody) and 12-amino acid peptide coated wells (in the absence of anti-P. falciparum monoclonal antibody) were used as controls. Controls showed 40-100 cpm.

13

## TABLE III

| Well No. | 1 | 2 | 3 | 4 | 5 |
|---|---|---|---|---|---|
| Unlabelled Antibody (micrograms/ml) | 10 | 0.1 | 0.01 | 0.001 | $1 \times 10^{-4}$ |

### Residual Radioactivity (cpm)

| | 1 | 2 | 3 | 4 | 5 |
|---|---|---|---|---|---|
| 2A10 | 1844 | 775 | 96 | 90 | 71 |
| 1E9 | 3787 | 2475 | 888 | 176 | 86 |
| 3D6 | 457 | 119 | 121 | 83 | 78 |
| 2C11 | 2874 | 1761 | 863 | 296 | 107 |

The controls in which the wells were incubated using dilutions of three other non-specific monoclonal antibodies of the same isotype resulted in residual radioactivity ranging between 44 and 100.

The above results show that several monoclonal anti-P. falciparum antibodies recognize and bind quantitatively to the synthetic 12-amino acid peptide.

EXAMPLE VIII

Immunization with the Synthetic Repeated Epitope of P. falciparum (12 MER and 24 MER)

A tandemly repeated peptide (3X and 6X) is synthesized as described above, except a cysteine residue is added at the N-terminus. To determine whether the synthesis had been performed correctly, an aliquot is subjected to acid hydrolysis at reduced pressure (5.6M HCl 110°C, 72 hours) and its, amino acid composition is determined. The peptide is coupled to a carrier protein (e.g. keyhole limpet hemocyanin, or tetanus toxoid, through its N terminal cysteine residue, using a m-malemidolbehzoyl-N-hydroxysuccinimide ester (MBS) as the coupling reagent (Ling et al, Biochemistry 18, 690 (1979)). This is a bifunctional reagent

14

which under appropriate conditions reacts with the amino group of the carrier and with the third group of the peptides. 4 mg of the carrier protein in 0.25 ml of 0.05 $PO_4$ buffer, pH 7.2, is reacted dropwise with 0.7 mg MBS dissolved in dimethyl formamide and stirred for 30 minutes at room temperature. The product MB carrier is separated from the unreacted chemicals by passage in a Sephadex C-25 column equilibrated in 0.05 M $PO_4$ buffer, pH 6.0. The MB carrier is then reacted with 5 mg of the 12- or 24-MER containing compound, dissolved in PBS (pH 7.4.) The mixture is stirred for 3 hours at room temperature and coupling is monitored with radioactive peptide. The conjugate is dialyzed and used as a vaccine for administration to non-human primates in a physiologically acceptable medium.

Alternatively, the tandemly repeated peptide (3X) can be further polymerized with glutaraldehyde as follows: Dissolve 20 mg of peptide in 10 ml of phosphate buffered saline (PBS). Make fresh glutaraldehyde from a stock with 13 milliliters of PBS. Stir the peptide and glutaraldehyde overnight at room temperature. Neutralize the excess glutaraldehyde with 1M ethanolamine. Separate the polymerized peptide by high performance liquid chromatography (HPLC) using sizing columns, and dialyze repeatedly against water. This is then used in a vaccine preparation.

Five chimpanzees are immunized with 200 micrograms of the conjugated protein or the polymerized product absorbed to aluminum hydroxide gel. Their serum is monitored for the presence of antibodies to CS proteins of P. falciparum using an immunoradiometric assay. Serum dilutions are incubated in antigen-coated wells of microtiter plates. The presence of chimpanzee antibody bound to the solid-phase antigen is monitored by incubation with [$^{125}$I]-labeled affinity-purified rabbit-anti-human IgG (which strongly cross-reacts with chimpanzee IgG).

After 30 days, the serum titer of the chimpanzees rises to titers of greater than 1/1000. At this time, these chimpanzees (as well as five other control chimpanzees injected with non-conjugated carrier protein adsorbed to aluminum hydroxide) are challenged with 2,000 viable P. falciparum sporozoites. The infection is monitored daily for a total of 30 days by microscopic examination of blood smears, starting one week after the inoculation of the parasites. The results show that the five chimpanzees immunized with the vaccine (conjugated protein) are totally protected, that is, no parasites are found in their blood. In contrast, the control chimpanzees have trophozoites of P. falciparum in their circulation 10-12 days after challenge. Based on the close similarities of human and chimpanzee immune responses and on the fact that protection immunity has been obtained in humans by injection of inactivated sporozoites of P. falciparum, the results obtained upon immunization of chimpanzees with the described synthetic peptide will also be obtained following similar treatment of human patients.

**Claims**
**Claims for the following Contracting States : BE, CH, DE, FR, GB, IT, NL, SE**

1. A peptide comprising the amino acid sequence (pro-asn-ala-asn) tandemly repeated at least 23 times.

2. The peptide according to Claim 1, wherein said amino acid sequence corresponds to an epitope of the CS protein of a sporozoite of the species Plasmodium falciparum.

3. The peptide according to Claim 1 or 2, wherein the peptide is a chemically synthesized peptide.

4. The peptide according to any of Claims 1 to 3, wherein the peptide is antigenic.

5. A vaccine against malaria comprising as an active ingredient the peptide of any of Claims 1 to 4 and a carrier.

6. The vaccine according to Claim 5 wherein said peptide is adsorbed or covalently attached to a carrier protein.

7. The vaccine according to any of Claims 5 and 6 further comprising a physiologically acceptable medium.

8. The vaccine according to any of Claims 5 to 7, wherein the vaccine is against P. falciparum sporozoites.

9. The vaccine according to any of Claims 5 to 8, wherein said active ingredient is immunochemically reactive with a monoclonal or polyclonal antibody to a sporozoite CS protein of the species P.

EP 0 187 858 B1

falciparum.

10. Use of the vaccine according to any of Claims 5 to 9 for preparing a medicament for immunizing a mammal against malaria.

11. A DNA fragment comprising a deoxynucleotide sequence coding for any of the peptides of Claims 1 and 2.

12. A recombinant DNA molecule comprising the DNA fragment of Claim 11.

13. The recombinant DNA molecule according to Claim 12, wherein said DNA fragment is inserted into a vector at a site suitable for expression of the coding sequence, either directly or as a fusion protein.

14. A microorganism transformed by the recombinant DNA molecule of any of Claims 12 and 13.

15. The microorganism of Claim 14 which is E. coli.

16. A method of preparing antibodies to CS antigen of P. falciparum sporozoites, said method comprising the immunization of a host using the peptide of any of Claims 1 to 4, said host being used only as a source for obtaining the antibodies.

17. A pharmaceutical composition for neutralizing the infectivity of P. falciparum sporozoites comprising a peptide according to any of Claims 1 to 4 coupled to a carrier.

**Claim for the following Contracting State AT**

1. A peptide comprising the amino acid sequence (pro-asn-ala-asn).

2. The peptide according to claim 1, wherein the sequence (pro-asn-ala-asn) is tandemly repeated 3 times.

3. The peptide according to claim 1, wherein the sequence (pro-asn-ala-asn) is tandemly repeated 6 times.

4. The peptide according to claim 1, wherein the sequence (pro-asn-ala-asn) is tandemly repeated at least 23 times.

5. The peptide according to any of claims 1 to 4, wherein said amino acid sequence corresponds to an epitope of the CS protein of a sporozoite of the species Plasmodium falciparum.

6. The peptide according to any of claims 1 to 5, wherein the peptide is a chemically synthesized peptide.

7. The peptide according to any of claims 1 to 6, wherein the peptide is antigenic.

8. A vaccine against malaria comprising as an active ingredient the peptide of any of claims 1 to 7 and a carrier.

9. The vaccine according to claim 8, wherein said peptide is adsorbed or covalently attached to a carrier protein.

10. The vaccine according to any of claims 8 and 9 further comprising a physiologically acceptable medium.

11. The vaccine according to any of claims 8 to 10, wherein the vaccine is against P. falciparum sporozoites.

12. The vaccine according to any of claims 8 to 11, wherein said active ingredient is immunochemically reactive with a monoclonal or polyclonal antibody to a sporozoite CS protein of the species P.

16

falciparum.

13. Use of the vaccine according to any of claims 8 to 12 for preparing a medicament for immunizing a mammal against malaria.

14. A DNA fragment comprising a deoxynucleotide sequence coding for any of the peptides of claims 1 to 5.

15. A recombinant DNA molecule comprising the DNA fragment of claim 14.

16. The recombinant DNA molecule according to claim 15, wherein said DNA fragment is inserted into a vector at a site suitable for expression of the coding sequence, either directly or as a fusion protein.

17. A microorganism transformed by the recombinant DNA molecule of any of claims 15 and 16.

18. The microorganism of claim 17 which is E. coli.

19. A method of preparing antibodies to CS antigen of P. falciparum sporozoites, said method comprising the immunization of a host using the peptide of any of claims 1 to 7, said host being used only as a source for obtaining the antibodies.

20. A method for preparing a pharmaceutical composition for neutralizing the infectivity of P. falciparum sporozoites comprising a peptide according to any of claims 1 to 7 coupled to a carrier by combining the components in a manner known per se.

21. A process for preparing a peptide comprising the amino acid sequence (pro-asn-ala-asn) by synthesizes or recombinant DNA technology.

22. The process according to claim 21, wherein the sequence (pro-asn-ala-asn) is tandemly repeated 3 times.

23. The process according to claim 21, wherein the sequence (pro-asn-ala-asn) is tandemly repeated 6 times.

24. The process according to claim 21, wherein the sequence (pro-asn-ala-asn) is tandemly repeated at least 23 times.

25. The process according to any of claims 21 to 24, wherein said amino acid sequence corresponds to an epitope of the CS protein of a sporozoite of the species Plasmodium falciparum.

26. The process according to any of claims 21 to 25, wherein the peptide is a chemically synthesized peptide.

27. The process according to any of claims 21 to 26, wherein the peptide is antigenic.

28. A process for preparing a vaccine against malaria comprising as an active ingredient the peptide of any of claims 1 to 7 and a carrier by coupling the peptide to the carrier.

29. The process according to claim 28, wherein said peptide is adsorbed or covalently attached to a carrier protein.

30. The process according to any of claims 28 and 29 further comprising the addition of a physiologically acceptable medium.

31. The process according to any of claims 28 to 30, wherein the vaccine is against P. falciparum sporozoites.

32. The process according to any of claims 28 to 31, wherein said active ingredient is immunochemically reactive with a monoclonal or polyclonal antibody to a sporozoite CS protein of the species P. falciparum.

33. Use of the vaccine according to any of claims 8 to 12 or prepared according to any of claims 28 to 32 for preparing a medicament for immunizing a mammal against malaria.

34. A process for preparing a DNA fragment comprising a deoxynucleotide sequence coding for any of the peptides of claims 1 to 5 or any of the peptides prepared according to claims 21 to 25 by recombinant DNA technology.

35. A process for preparing a recombinant DNA molecule comprising the DNA fragment of claim 14 or the DNA fragment prepared according to claim 34 by recombinant DNA technology.

36. The process according to claim 35, wherein said DNA fragment is inserted into a vector at a site suitable for expression of the coding sequence, either directly or as a fusion protein.

37. A process for preparing a microorganism transformed by the recombinant DNA molecule of any of claims 15 and 16 or the DNA molecule prepared according to any of claims 35 and 36 by transforming a host cell in a manner known per se.

38. The process of claim 37, wherein the microorganism is E. coli.

**Claim for the following Contracting State : LU**

1. A peptide comprising the amino acid sequence (pro-asn-ala-asn).

2. The peptide according to Claim 1, wherein the sequence (pro-asn-ala-asn) is tandemly repeated 3 times.

3. The peptide according to Claim 1, wherein the sequence (pro-asn-ala-asn) is tandemly repeated 6 times.

4. The peptide according to Claim 1, wherein the sequence (pro-asn-ala-asn) is tandemly repeated at least 23 times.

5. The peptide according to any of Claims 1 to 4, wherein said amino acid sequence corresponds to an epitope of the CS protein of a sporozoite of the species Plasmodium falciparum.

6. The peptide according to any of Claims 1 to 5, wherein the peptide is a chemically synthesized peptide.

7. The peptide according to any of Claims 1 to 6, wherein the peptide is antigenic.

8. A vaccine against malaria comprising as an active ingredient the peptide of any of Claims 1 to 7 and a carrier.

9. The vaccine according to Claim 8, wherein said peptide is adsorbed or covalently attached to a carrier protein.

10. The vaccine according to any of Claims 8 and 9 further comprising a physiologically acceptable medium.

11. The vaccine according to any of Claims 8 to 10, wherein the vaccine is against P. falciparum sporozoites.

12. The vaccine according to any of Claims 8 to 11, wherein said active ingredient is immunochemically reactive with a monoclonal or polyclonal antibody to a sporozoite CS protein of the species P. falciparum.

13. Use of the vaccine according to any of Claims 8 to 12 for preparing a medicament for immunizing a mammal against malaria.

14. A DNA fragment comprising a deoxynucleotide sequence coding for any of the peptides of Claims 1 to 5.

15. A recombinant DNA molecule comprising the DNA fragment of Claim 14.

16. The recombinant DNA molecule according to Claim 15, wherein said DNA fragment is inserted into a vector at a site suitable for expression of the coding sequence, either directly or as a fusion protein.

17. A microorganism transformed by the recombinant DNA molecule of any of Claims 15 and 16.

18. The microorganism of Claim 17 which is E. coli.

19. A method of preparing antibodies to CS antigen of P. falciparum sporozoites, said method comprising the immunization of a host using the peptide of any of Claims 1 to 7, said host being used only as a source for obtaining the antibodies.

20. A pharmaceutical composition for neutralizing the infectivity of P. falciparum sporozoites comprising a peptide according to any of Claims 1 to 7 coupled to a carrier.

**Revendications**
**Revendications pour les Etats contractants suivants : BE, CH, DE, FR, GB, IT, NL, SE**

1. Peptide comprenant la séquence d'amino-acides (pro-asn-ala-asn) répétée au moins 23 fois en tandem.

2. Peptide selon la revendication 1, dans lequel ladite séquence d'amino-acides correspond à un épitope de la protéine CS d'un sporozoïte de l'espèce Plasmodium falciparum.

3. Peptide selon la revendication 1 ou 2, ledit peptide étant un peptide obtenu par synthèse chimique.

4. Peptide selon l'une quelconque des revendications 1 à 3, ledit peptide étant antigénique.

5. Vaccin contre la malaria comprenant comme ingrédient actif le peptide selon l'une quelconque des revendications 1 à 4 et un support.

6. Vaccin selon la revendication 5, caractérisé en ce que ledit peptide est adsorbé ou fixé par liaison covalente à une protéine de support.

7. Vaccin selon l'une quelconque des revendications 5 et 6, comprenant en outre un milieu physiologiquement acceptable.

8. Vaccin selon l'une quelconque des revendications 5 à 7, dans lequel le vaccin agit contre les sporozoïtes de P. falciparum.

9. Vaccin selon l'une quelconque des revendications 5 à 8, dans lequel ledit ingrédient actif réagit immunochimiquement avec un anticorps monoclonal ou polyclonal contre une protéine CS de sporozoïte de l'espèce P. falciparum.

10. Utilisation du vaccin sel on l'une quelconque des revendications 5 à 9 pour préparer un médicament destiné à immuniser un mammifère contre la malaria.

11. Fragment d'ADN comprenant une séquence de désoxynucléotides codant pour l'un quelconque des peptides des revendications 1 et 2.

12. Molécule d'ADN recombinant comprenant le fragment d'ADN de la revendication 11.

EP 0 187 858 B1

**13.** Molécule d'ADN recombinant selon la revendication 12, dans laquelle ledit fragment d'ADN est inséré dans un vecteur en un site approprié pour l'expression de la séquence codante, directement ou sous la forme d'une protéine de fusion.

**14.** Micro-organisme transformé par la molécule d'ADN recombinant de l'une quelconque des revendications 12 et 13.

**15.** Micro-organisme selon la revendication 14, qui est E. coli.

**16.** Procédé de préparation d'anticorps vis-à-vis de l'antigène CS de sporozoïtes de P. falciparum, ledit procédé comprenant l'immunisation d'un hôte en utilisant le peptide selon l'une quelconque des revendications 1 à 4, ledit hôte étant utilisé uniquement comme source pour l'obtention des anticorps.

**17.** Composition pharmaceutique pour neutraliser le pouvoir infectieux des sporozoïtes de P. falciparum, comprenant un peptide selon l'une quelconque des revendications 1 à 4, couplé à un support.

**Revendications pour l'Etat contractant suivant : AT**

**1.** Peptide comprenant la séquence d'amino-acides (pro-asn-ala-asn).

**2.** Peptide selon la revendication 1, dans lequel la séquence (pro-asn-ala-asn) est répétée 3 fois en tandem.

**3.** Peptide selon la revendication 1, dans lequel la séquence (pro-asn-ala-asn) est répétée 6 fois en tandem.

**4.** Peptide selon la revendication 1, dans lequel la séquence (pro-asn-ala-asn) est répétée au moins 23 fois en tandem.

**5.** Peptide selon l'une quelconque des revendications 1 à 4, dans lequel ladite séquence d'amino-acides correspond à un épitope de la protéine CS d'un sporozoïte de l'espèce Plasmodium falciparum.

**6.** Peptide selon l'une quelconque des revendications 1 à 5, ledit peptide étant un peptide obtenu par synthèse chimique.

**7.** Peptide selon l'une quelconque des revendications 1 à 6, ledit peptide étant antigénique.

**8.** Vaccin contre la malaria comprenant comme ingrédient actif le peptide selon l'une quelconque des revendications 1 à 7 et un support.

**9.** Vaccin selon la revendication 8, dans lequel ledit peptide est adsorbé ou lié par liaison covalente à une protéine de support.

**10.** Vaccin selon l'une quelconque des revendications 8 et 9, comprenant en outre un milieu physiologiquement acceptable.

**11.** Vaccin selon l'une quelconque des revendications 8 à 10, dans lequel ledit vaccin agit contre les sporozoïtes de P. falciparum.

**12.** Vaccin selon l'une quelconque des revendications 8 à 11, dans lequel ledit ingrédient actif réagit immunochimiquement avec un anticorps monoclonal ou polyclonal contre une protéine CS de sporozoïte de l'espèce P. falciparum.

**13.** Utilisation du vaccin selon l'une quelconque des revendications 8 à 12 pour préparer un médicament destiné à immuniser un mammifère contre la malaria.

**14.** Fragment d'ADN comprenant une séquence de désoxynucléotides codant pour l'un quelconque des peptides des revendications 1 à 5.

20

**15.** Molécule d'ADN recombinant comprenant le fragment d'ADN de la revendication 14.

**16.** Molécule d'ADN recombinant selon la revendication 15, dans laquelle ledit fragment d'ADN est inséré dans un vecteur en un site approprié pour l'expression de la séquence codante, directement ou sous la forme d'une protéine de fusion.

**17.** Micro-organisme transformé par la molécule d'ADN recombinant de l'une quelconque des revendications 15 et 16.

**18.** Micro-organisme selon la revendication 17, qui est E. coli.

**19.** Procédé de préparation d'anticorps vis-à-vis de l'antigène CS de sporozoïtes de P. falciparum, ledit procédé comprenant l'immunisation d'un hôte en utilisant le peptide selon l'une quelconque des revendications 1 à 7, ledit hôte étant utilisé uniquement comme source pour l'obtention des anticorps.

**20.** Procédé pour préparer une composition pharmaceutique pour neutraliser le pouvoir infectieux de sporozoïtes de P. falciparum, comprenant un peptide selon l'une quelconque des revendications 1 à 7 couplé à un support.

**21.** Procédé pour préparer un peptide comprenant la séquence d'amino-acides (pro-asn-ala-asn) par synthèse ou par technologie de l'ADN recombinant.

**22.** Procédé selon la revendication 21, dans lequel la séquence (pro-asn-ala-asn) est répétée 3 fois en tandem.

**23.** Procédé selon la revendication 21, dans lequel la séquence (pro-asn-ala-asn) est répétée 6 fois en tandem.

**24.** Procédé selon la revendication 21, dans lequel la séquence (pro-asn-ala-asn) est répétée au moins 23 fois en tandem.

**25.** Procédé selon l'une quelconque des revendications 21 à 24, dans lequel ladite séquence d'amino-acides correspond à un épitope de la protéine CS d'un sporozoïte de l'espèce Plasmodium falciparum.

**26.** Procédé selon l'une quelconque des revendications 21 à 25, dans lequel le peptide est un peptide obtenu par synthèse chimique.

**27.** Procédé selon l'une quelconque des revendications 21 à 26, dans lequel ledit peptide est antigénique.

**28.** Procédé pour préparer un vaccin contre la malaria comprenant comme ingrédient actif le peptide selon l'une quelconque des revendications 1 à 7 et un support, en couplant le peptide au support.

**29.** Procédé selon la revendication 28, dans lequel le peptide est adsorbé ou fixé par liaison covalente à une protéine de support.

**30.** Procédé selon l'une quelconque des revendications 28 et 29, comprenant en outre l'addition d'un milieu physiologiquement acceptable.

**31.** Procédé selon l'une quelconque des revendications 28 à 30, dans lequel le vaccin agit contre des sporozoïtes de P. falciparum.

**32.** Procédé selon l'une quelconque des revendications 28 à 31, dans lequel ledit ingrédient actif réagit immunochimiquement avec un anticorps monoclonal ou polyclonal contre une protéine CS de sporozoïte de l'espèce P. falciparum.

**33.** Utilisation du vaccin selon l'une quelconque des revendications 28 à 32 pour préparer un médicament destiné à immuniser un mammifère contre la malaria.

EP 0 187 858 B1

**34.** Procédé de préparation d'un fragment d'ADN comprenant une séquence de désoxynucléotides codant pour l'un quelconque des peptides des revendications 1 à 5 ou de l'un quelconque des peptides préparés selon les revendications 21 à 25 par technologie de l'ADN recombinant.

**35.** Procédé pour préparer une molécule d'ADN recombinant comprenant le fragment d'ADN de la revendication 14 ou le fragment d'ADN préparé selon la revendication 34 par technologie de l'ADN recombinant.

**36.** Procédé selon la revendication 35, dans lequel ledit fragment d'ADN est inséré dans un vecteur en un site approprié pour l'expression de la séquence codante, directement ou sous la forme d'une protéine de fusion.

**37.** Procédé pour préparer un micro-organisme transformé par la molécule d'ADN recombinant selon l'une quelconque des revendications 15 et 16 ou la molécule d'ADN préparée selon l'une quelconque des revendication 35 et 36 en transformant une cellule-hôte d'une façon connue en soi.

**38.** Procédé selon la revendication 37, dans lequel le micro-organisme est E. coli.

**Revendications pour l'Etat contractant suivant : LU**

**1.** Peptide comprenant la séquence d'amino-acides (pro-asn-ala-asn).

**2.** Peptide selon la revendication 1, dans lequel la séquence (pro-asn-ala-asn) est répétée 3 fois en tandem.

**3.** Peptide selon la revendication 1, dans lequel la séquence (pro-asn-ala-asn) est répétée 6 fois en tandem.

**4.** Peptide selon la revendication 1, dans lequel la séquence (pro-asn-ala-asn) est répétée au moins 23 fois en tandem.

**5.** Peptide selon l'une quelconque des revendications 1 à 4, dans lequel ladite séquence d'amino-acides correspond à un épitope de la protéine CS d'un sporozoïte de l'espèce Plasmodium falciparum.

**6.** Peptide selon l'une quelconque des revendications 1 à 5, ledit peptide étant un peptide obtenu par synthèse chimique.

**7.** Peptide selon l'une quelconque des revendications 1 à 6, ledit peptide étant antigénique.

**8.** Vaccin contre la malaria comprenant comme ingrédient actif le peptide selon l'une quelconque des revendications 1 à 7 et un support.

**9.** Vaccin selon la revendication 8, dans lequel ledit peptide est adsorbé ou fixé par liaison covalente à une protéine de support.

**10.** Vaccin selon l'une quelconque des revendications 8 et 9, comprenant en outre un milieu physiologiquement acceptable.

**11.** Vaccin selon l'une quelconque des revendications 8 à 10, dans lequel ledit vaccin agit contre les sporozoïtes de P. falciparum.

**12.** Vaccin selon l'une quelconque des revendications 8 à 11, dans lequel ledit ingrédient actif réagit immunochimiquement avec un anticorps monoclonal ou polyclonal contre une protéine CS de sporozoïte de l'espèce P. falciparum.

**13.** Utilisation du vaccin selon l'une quelconque des revendications 8 à 12 pour préparer un médicament destiné à immuniser un mammifère contre la malaria.

22

EP 0 187 858 B1

**14.** Fragment d'ADN comprenant une séquence de désoxynucléotides codant pour l'un quelconque des peptides des revendications 1 à 5.

**15.** Molécule d'ADN recombinant comprenant le fragment d'ADN de la revendication 14.

**16.** Molécule d'ADN recombinant selon la revendication 15, dans laquelle ledit fragment d'ADN est inséré dans un vecteur en un site approprié pour l'expression de la séquence codante, directement ou sous la forme d'une protéine de fusion.

**17.** Micro-organisme transformé par la molécule d'ADN recombinant de l'une quelconque des revendications 15 et 16.

**18.** Micro-organisme selon la revendication 17, qui est E. coli.

**19.** Procédé de préparation d'anticorps vis-à-vis de l'antigène CS de sporozoïtes de P. falciparum, ledit procédé comprenant l'immunisation d'un hôte en utilisant le peptide selon l'une quelconque des revendications 1 à 7, ledit hôte étant utilisé uniquement comme source pour l'obtention des anticorps.

**20.** Composition pharmaceutique pour neutraliser le pouvoir infectieux de sporozoïtes de P. falciparum, comprenant un peptide selon l'une quelconque des revendications 1 à 7 couplé à un support.

**Patentansprüche**
**Patentansprüche für folgende Vertragsstaaten : BE, CH, DE, FR, GB, IT, NL, SE**

**1.** Ein Peptid enthaltend die Aminosäuresequenz (pro-asn-ala-asn) mindestens 23 mal hintereinander wiederholt.

**2.** Das Peptid nach Anspruch 1, worin die Aminosäuresequenz einem Epitop des CS-Proteins eines Sporozoiten der Art Plasmodium falciparum entspricht.

**3.** Das Peptid nach Anspruch 1 oder 2, worin das Peptid ein chemisch synthetisiertes Peptid ist.

**4.** Das Peptid nach einem der Ansprüche 1 bis 3, worin das Peptid antigen ist.

**5.** Ein Impfstoff gegen Malaria umfassend als Wirkstoff das Peptid nach einem der Ansprüche 1 bis 4 und einen Träger.

**6.** Der Impfstoff nach Anspruch 5, worin das Peptid an ein Trägerprotein adsorbiert oder kovalent angebracht ist.

**7.** Der Impfstoff nach einem der Ansprüche 5 und 6 weiterhin umfassend ein physiologisch akzeptables Medium.

**8.** Der Impfstoff nach einem der Ansprüche 5 bis 7, worin der Impfstoff gegen P. falciparum Sporozoiten gerichtet ist.

**9.** Der Impfstoff nach einem der Ansprüche 5 bis 8, worin der Wirkstoff mit einem monoclonalen oder polyclonalen Antikörper gegen ein Sporozoiten-CS-Protein der Art P. falciparum immunchemisch reaktionsfähig ist.

**10.** Verwendung des Impfstoffs nach einem der Ansprüche 5 bis 9 zum Herstellen eines Medikaments zum Immunisieren eines Säugers gegen Malaria.

**11.** Ein DNA-Fragment umfassend eine Desoxynucleotid-Sequenz, die für eines der Peptide der Ansprüche 1 und 2 codiert.

**12.** Ein rekombinantes DNA-Molekül umfassend das DNA-Fragment von Anspruch 11.

23

13. Das rekombinante DNA-Molekül nach Anspruch 12, worin das DNA-Fragment in einen Vektor an einer geeigneten Stelle zur Expression der codierenden Sequenz, entweder direkt oder als Fusionsprotein, eingefügt ist.

14. Ein durch das rekombinante DNA-Molekül nach einem der Ansprüche 12 und 13 transformierter Mikroorganismus.

15. Der Mikroorganismus nach Anspruch 14, der E. coli ist.

16. Ein Verfahren zum Herstellen von Antikörpern gegen CS-Antigen von P. falciparum Sporozoiten, wobei das Verfahren die Immunisierung eines Wirts unter Verwendung des Peptids nach einem der Ansprüche 1 bis 4 umfaßt, wobei der Wirt nur als Quelle zum Erhalten der Antikörper verwendet wird.

17. Eine pharmazeutische Zusammensetzung zum Neutralisieren der Infektiosität von P. falciparum Sporozoiten umfassend ein Peptid nach einem der Ansprüche 1 bis 4, gekoppelt an einen Träger.

**Patentansprüche für folgenden Vertragsstaat : AT**

1. Ein Peptid umfassend die Aminosäuresequenz (pro-asn-ala-asn).

2. Das Peptid nach Anspruch 1, worin die Sequenz (pro-asn-ala-asn) 3 mal hintereinander wiederholt ist.

3. Das Peptid nach Anspruch 1, worin die Sequenz (pro-asn-ala-asn) 6 mal hintereinander wiederholt ist.

4. Das Peptid nach Anspruch 1, worin die Sequenz (pro-asn-ala-asn) mindestens 23 mal hintereinander wiederholt ist.

5. Das Peptid nach einem der Ansprüche 1 bis 4, worin die Aminosäuresequenz einem Epitop des CS-Proteins eines Sporozoiten der Art Plasmodium falciparum entspricht.

6. Das Peptid nach einem der Ansprüche 1 bis 5, worin das Peptid ein chemisch synthetisiertes Peptid ist.

7. Das Peptid nach einem der Ansprüche 1 bis 6, worin das Peptid antigen ist.

8. Ein Impfstoff gegen Malaria umfassend als Wirkstoff das Peptid nach einem der Ansprüche 1 bis 7 und einen Träger.

9. Der Impfstoff nach Anspruch 8, worin das Peptid an ein Trägerprotein adsorbiert oder kovalent angebracht ist.

10. Der Impfstoff nach einem der Ansprüche 8 und 9 weiterhin umfassend ein physiologisch akzeptables Medium.

11. Der Impfstoff nach einem der Ansprüche 8 bis 10, worin der Impfstoff gegen P. falciparum Sporozoiten gerichtet ist.

12. Der Impfstoff nach einem der Ansprüche 8 bis 11, worin der Wirkstoff mit einem monoclonalen oder polyclonalen Antikörper gegen ein Sporozoiten-CS-Protein der Art P. falciporum immunchemisch reaktionsfähig ist.

13. Verwendung des Impfstoffs nach einem der Ansprüche 8 bis 12 zum Herstellen eines Medikaments zum Immunisieren eines Säugers gegen Malaria.

14. Ein DNA-Fragment umfassend eine Desoxynucleotid-Sequenz, die für eines der Peptide der Ansprüche 1 bis 5 codiert.

15. Ein rekombinantes DNA-Molekül umfassend das DNA-Fragment von Anspruch 14.

**16.** Das rekombinante DNA-Molekül nach Anspruch 15, worin das DNA-Fragment in einen Vektor an einer geeigneten Stelle zur Expression der codierenden Sequenz, entweder direkt oder als Fusionsprotein, eingefügt ist.

**17.** Ein durch das rekombinante DNA-Molekül nach einem der Ansprüche 15 und 16 transformierter Mikroorganismus.

**18.** Der Mikroorganismus nach Anspruch 17, der E. coli ist.

**19.** Ein Verfahren zum Herstellen von Antikörpern gegen CS-Antigen von P. falciparum Sporozoiten, wobei das Verfahren die Immunisierung eines Wirts unter Verwendung des Peptids nach einem der Ansprüche 1 bis 7 umfaßt, wobei der Wirt nur als Quelle zum Erhalten der Antikörper verwendet wird.

**20.** Ein Verfahren zum Herstellen einer pharmazeutischen Zusammensetzung zum Neutralisieren der Infektiosität von P. falciparum Sporozoiten umfassend ein Peptid nach einem der Ansprüche 1 bis 7, gekoppelt an einen Träger, durch Kombinieren der Bestandteile in an sich bekannter Weise.

**21.** Ein Verfahren zum Herstellen eines Peptids, umfassend die Aminosäuresequenz (pro-asn-ala-asn) durch Synthese oder rekombinante DNA-Technologie.

**22.** Das Verfahren nach Anspruch 21, worin die Sequenz (pro-asn-ala-asn) 3 mal hintereinander wiederholt ist.

**23.** Das Verfahren nach Anspruch 21, worin die Sequenz (pro-asn-ala-asn) 6 mal hintereinander wiederholt ist.

**24.** Das Verfahren nach Anspruch 21, worin die Sequenz mindestens 23 mal hintereinander wiederholt ist.

**25.** Das Verfahren nach einem der Ansprüche 21 bis 24, worin die Aminosäuresequenz einem Epitop des CS-Proteins eines Sporozoiten der Art Plasmodium falciparum entspricht.

**26.** Das Verfahren nach einem der Ansprüche 21 bis 25, worin das Peptid ein chemisch synthetisiertes Peptid ist.

**27.** Das Verfahren nach einem der Ansprüche 21 bis 26, worin das Peptid antigen ist.

**28.** Ein Verfahren zum Herstellen eines Impfstoffs gegen Malaria umfassend als einen Wirkstoff das Peptid nach einem der Ansprüche 1 bis 7 und einen Träger durch Kuppeln des Peptids an den Träger.

**29.** Das Verfahren nach Anspruch 28, worin das Peptid an ein Trägerprotein adsorbiert oder kovalent angefügt ist.

**30.** Das Verfahren nach einem der Ansprüche 28 und 29 weiterhin umfassend das Zusetzen eines physiologisch akzeptablen Mediums.

**31.** Das Verfahren nach einem der Ansprüche 28 bis 30, worin der Impfstoff gegen P. falciparum Sporozoiten gerichtet ist.

**32.** Das Verfahren nach einem der Ansprüche 28 bis 31, worin der Wirkstoff immunchemisch reaktionsfähig ist mit einem monoclonalen oder polyclonalen Antikörper gegen ein Sporozoiten-CS-Protein der Art P. falciporum.

**33.** Verwendung des Impfstoffs nach einem der Ansprüche 8 bis 12 oder hergestellt nach einem der Ansprüche 28 bis 32 zum Herstellen eines Medikaments zum Immunisieren eines Säugers gegen Malaria.

**34.** Ein Verfahren zum Herstellen eines DNA-Fragments umfassend eine Desoxynucleotid-Sequenz, die für eines der Peptide der Ansprüche 1 bis 5 oder eines der Peptide hergestellt nach den Ansprüchen 21 bis 25 codiert, durch rekombinante DNA-Technologie.

**35.** Ein Verfahren zum Herstellen eines rekombinanten DNA-Moleküls umfassend das DNA-Fragment von Anspruch 14 oder das DNA-Fragment hergestellt nach Anspruch 34 durch rekombinante DNA-Technologie.

**36.** Das Verfahren nach Anspruch 35, worin das DNA-Fragment in einen Vektor an einer geeigneten Stelle zur Expression der codierenden Sequenz, entweder direkt oder als Fusionsprotein, eingefügt wird.

**37.** Ein Verfahren zum Herstellen eines Mikroorganismus, der durch das rekombinante DNA-Molekül nach einem der Ansprüche 15 oder 16 oder das DNA-Molekül hergestellt nach einem der Ansprüche 35 und 36 transformiert ist, durch Transformieren einer Wirtszelle in an sich bekannter Weise.

**38.** Das Verfahren von Anspruch 37, worin der Mikroorganismus E. coli ist.

**Patentansprüche für folgenden Vertragsstaat : LU**

**1.** Ein Peptid umfassend die Aminosäuresequenz (pro-asn-ala-asn).

**2.** Das Peptid nach Anspruch 1, worin die Sequenz (pro-asn-ala-asn) 3 mal hintereinander wiederholt ist.

**3.** Das Peptid nach Anspruch 1, worin die Sequenz (pro-asn-ala-asn) 6 mal hintereinander wiederholt ist.

**4.** Das Peptid nach Anspruch 1, worin die Sequenz (pro-asn-ala-asn) mindestens 23 mal hintereinander wiederholt ist.

**5.** Das Peptid nach einem der Ansprüche 1 bis 4, worin die Aminosäuresequenz einem Epitop des CS-Proteins eines Sporozoiten der Art Plasmodium falciparum entspricht.

**6.** Das Peptid nach einem der Ansprüche 1 bis 5, worin das Peptid ein chemisch synthetisiertes Peptid ist.

**7.** Das Peptid nach einem der Ansprüche 1 bis 6, worin das Peptid antigen ist.

**8.** Ein Impfstoff gegen Malaria umfassend als Wirkstoff das Peptid nach einem der Ansprüche 1 bis 7 und einen Träger.

**9.** Der Impfstoff nach Anspruch 8, worin das Peptid an ein Trägerprotein adsorbiert oder kovalent angebracht ist.

**10.** Der Impfstoff nach einem der Ansprüche 8 und 9 weiterhin umfassend ein physiologisch akzeptables Medium.

**11.** Der Impfstoff nach einem der Ansprüche 8 bis 10, worin der Impfstoff gegen P. falciparum Sporozoiten gerichtet ist.

**12.** Der Impfstoff nach einem der Ansprüche 8 bis 11, worin der Wirkstoff mit einem monoclonalen oder polyclonalen Antikörper gegen ein Sporozoiten-CS-Protein der Art P. falciparum immunchemisch reaktionsfähig ist.

**13.** Verwendung des Impfstoffs nach einem der Ansprüche 8 bis 12 zum Herstellen eines Medikaments zum Immunisieren eines Säugers gegen Malaria.

**14.** Ein DNA-Fragment umfassend eine Desoxynucleotid-Sequenz, die für eines der Peptide der Ansprüche 1 bis 5 codiert.

**15.** Ein rekombinantes DNA-Molekül umfassend das DNA-Fragment von Anspruch 14.

**16.** Das rekombinante DNA-Molekül nach Anspruch 15, worin das DNA-Fragment in einen Vektor an einer geeigneten Stelle zur Expression der codierenden Sequenz, entweder direkt oder als Fusionsprotein, eingefügt ist.

**17.** Ein durch das rekombinante DNA-Molekül nach einem der Ansprüche 15 und 16 transformierter Mikroorganismus.

**18.** Der Mikroorganismus nach Anspruch 17, der E. coli ist.

**19.** Ein Verfahren zum Herstellen von Antikörpern gegen CS-Antigen von P. falciparum Sporozoiten, wobei das Verfahren die Immunisierung eines Wirts unter Verwendung des Peptids nach einem der Ansprüche 1 bis 7 umfaßt, wobei der Wirt nur als Quelle zum Erhalten der Antikörper verwendet wird.

**20.** Eine pharmazeutische Zusammensetzung zum Neutralisieren der Infektiosität von P. falciparum Sporozoiten umfassend eine Peptid nach einem der Ansprüche 1 bis 7, gekoppelt an einen Träger.

```
        Pro   Asn   Ala   Asn
G¹⁵     CCA   AAT   GCA   AAC
         C     T     A     C
         A     C     A     C
         C     T     A     T
         T     T     A     C
         C     T     A     T
         T     T     A     T
         T     T     C     T
         A     T     A     T
         T     T     A     C
         C     T     A     T
         T     T     A     T
         T     T     C     T
         A     T     A     T
         A     T     A     C
         A     C     A     C
         C     T     A     T
         T     T     C     T
         A     T     A     T
         A     T     A     C
         A     T     A     C
         A     T     A     C
         C     T     A     T
        CCT   AAT   AAA   AAC
        AAT   CAA   GCC   CCC   C¹⁸
```

# FIG. I

FIG. 2A

FIG. 2B